# EUROPÄISCHE PATENTANMELDUNG

(11) **EP 1 795 225 A1**
(43) Veröffentlichungstag der Anmeldung: **13.06.2007**
(21) Anmeldenummer: 06022974.7
(22) Anmeldetag: 04.11.2006
(51) Int. Cl.: A61N 1/375

(54) **Gehäuse für ein medizinisches Implantat**

(30) Priorität: 08.12.2005 DE 102005058551; 24.01.2006 DE 102006003223
(71) Anmelder: BIOTRONIK CRM Patent AG, 6341 Baar (CH)
(72) Erfinder: Starke, Marcel, 12167 Berlin (DE); Litzke, Jan, 13507 Berlin (DE)
(74) Vertreter: Lindner-Vogt, Karin L.

(57) **Zusammenfassung**

Gehäuse für ein medizinisches Implantat, wie einen Herzschrittmacher, Defibrillator, Kardioverter oder dergleichen, wobei das Gehäuse ein Hohlgehäuse und ein an dem Hohlgehäuse befestigtes Anschlussgehäuse aufweist, das wenigstens einen in einer von außen zugänglichen Kavität des Anschlussgehäuses angeordneten elektrischen Anschluss zum Anschließen einer Elektrodenleitung besitzt.

Das Anschlussgehäuse ist mit dem Hohlgehäuse mittels eines Klebstoffs verklebt, wobei der Kunststoff des Anschlussgehäuses Polyurethan und/oder Polycarbonat enthält und der Klebstoff ein Epoxid oder Polyurethan ist.

## Beschreibung

Die Erfindung betrifft ein Gehäuse für ein medizinisches Implantat wie beispielsweise einen implantierbaren Herzschrittmacher, Defibrillator, Kardioverter oder dergleichen. Das Gehäuse umfasst ein Hohlgehäuse, welches beispielsweise der Aufnahme von Steuerelektronik, Kondensatoren und Batterien dient. Außerdem umfasst das Gehäuse einen Anschlusskörper, der an dem Hohlgehäuse befestigt ist und wenigstens eine, in der Regel aber zwei bis vier in einer oder mehreren von außen zugänglichen Kavitäten des Anschlusskörpers angeordnete elektrische Anschlüsse aufweist, die zum Anschließen einer oder mehrerer Elektrodenleitungen dienen. Ein solcher Anschlusskörper wird auch als Header bezeichnet. Hohlgehäuse und Header ergeben zusammen ein hermetisch dichtes Gehäuse, welches im Inneren des Hohlgehäuses die genannten elektrischen Komponenten aufweist, die über Zuleitungen mit dem elektrischen Anschluss oder den elektrischen Anschlüssen in dem Header verbunden sind.

Das Hohlgehäuse ist in der Regel aus biokompatiblem Metall gefertigt. Die elektrische Verbindung der elektrischen Komponenten in dem Hohlgehäuse zu den im Header befindlichen elektrischen Zuleitungen zu den elektrischen Anschlüssen erfolgt in der Regel über Durchführungen, die in eine Wand des Hohlgehäuses eingelassen sind und einerseits das Hohlgehäuse hermetisch dicht abschließen und andererseits auch als Filterdurchführungen ausgebildet sein können, die eine elektrische Tiefpasswirkung haben.

Das Anschlussgehäuse (auch Header genannt) besteht in der Regel aus transparenten, isolierendem Kunststoff. Die elektrischen Anschlüsse sind als Buchsen ausgeführt, die entsprechende Stecker von Elektrodenleitungen aufnehmen können. Aufgrund der Transparenz des Anschlussgehäuses ist von außen sichtbar, ob ein Elektrodenleitungsstecker weit genug in die Buchse eines jeweiligen elektrischen Anschlusses eingeführt ist. Das Anschlussgehäuse kann einen Basiskörper und/oder einen Anschlusskörper umfassen, die in geeigneter Weise miteinander verbunden sind. Es ist auch jeglicher anderer aus dem Stand der Technik bekannter Aufbau möglich, insbesondere ein mehrteiliger Aufbau, bei dem die beinhalteten Komponenten einzeln zusammengefügt sind. Im Folgenden wird daher nur noch von Anschlussgehäuse gesprochen.

An die Qualität eines Gehäuses für ein medizinisches Implantat werden hohe Anforderungen gestellt. Insbesondere müssen Hohlgehäuse und Anschlussgehäuse über Jahre hinweg zuverlässig und dicht zusammenwirken. Das Anschlussgehäuse selbst muss über lange Zeit stabil und passgenau sein.

Um die vorgenannten Anforderungen zu erfüllen, sind aus dem Stand der Technik verschiedene Ansätze bekannt. Zum einen ist es bekannt, das Anschlussgehäuse an das Hohlgehäuse direkt anzugießen. Dazu wird das Hohlgehäuse mit daran befestigten Zuleitungen für die elektrischen Anschlüsse und Kontaktbuchsen für die Elektrodenleitungsstecker in eine Gussform eingesetzt und ein Basiskörper durch Füllen der geschlossenen Gussform mit flüssigen Kunststoff umgossen. Der flüssige Kunststoff wird in der Gussform aushärten gelassen und ergibt im Ergebnis - nach Entfernen der Gussform - einen in einem Arbeitsschritt hergestellten, unmittelbar fest mit dem Hohlgehäuse verbundenes Anschlussgehäuse.

Alternativ sind vormontierte Anschlussgehäuse bekannt, bei denen die elektrischen Bestandteile wie beispielsweise elektrische Kontaktbuchsen oder elektrische Zuleitungen zunächst in vorgefertigte Spritzgussteile aus Kunststoff eingesetzt werden. Solche mehrteiligen, vormontierbaren Anschlussgehäuse sind beispielsweise aus WO 01/99239, EP 0 429 024, US 5,282,841 oder US 6,205,358 bekannt.

Aufgabe der Erfindung ist es, ein Gehäuse für ein elektromedizinisches Implantat wie einen Herzschrittmacher oder dergleichen zu schaffen, welches eine möglichst einfache, wenig fehlerträchtige und kostengünstige Herstellung erlaubt und zu einem Gehäuse mit den geforderten Eigenschaften führt.

Erfindungsgemäß wird diese Aufgabe durch ein Gehäuse der eingangs genannten Art erreicht, wobei der Kunststoff für das Anschlussgehäuse Polyurethan und/oder Polykarbonat enthält oder zumindest zu wesentlichen Teilen aus Polyurethan und/oder Polykarbonat besteht und der Klebstoff schnellhärtend ist. Bevorzugt ist das Anschlussgehäuse transparent und erlaubt einen Blick von außen auf den elektrischen Anschluss.

Bevorzugt ist der Klebstoff während einer Zeit von 1 bis 15 Minuten, besonders bevorzugt von 5 bis 10 Minuten weiterverarbeitbar.

Vorzugsweise härtet der Klebstoff bei Temperaturen von ungefähr 20° bis ungefähr 85° Celsius aus.

Als besonders bevorzugte Werkstoffe für das Anschlussgehäuse haben sich Polyurethan, vorzugsweise aromatisches und/oder aliphatisches Acryl-Polyester Polyurethan, oder Epoxidharz erwiesen. Diese Materialien erfüllen die gewünschten Anforderungen an Langzeitstabilität, Transparenz und Maßhaltigkeit und lassen sich insbesondere auch gut mit Epoxid und/oder Polyurethan verkleben.

Der Vorteil von Epoxid - umfassend insbesondere Epoxidharz - als Klebstoff ist einmal durch günstige Verarbeitungseigenschaften des Epoxid und zum anderen durch die mit Epoxid erzielbare hohe Festigkeit der Klebverbindung und durch die Biokompatibilität von Epoxid gegeben. In Bezug auf die erzielbare Festigkeit der Klebverbindung ist es besonders vorteilhaft, dass Epoxid sowohl sehr gut an verschiedenen Oberflächen haftet, also große Adhäsionskräfte erlaubt, als auch selbst eine gute innere Zugfestigkeit besitzt, also große Kohäsionskräfte entwickelt.

Vorzugsweise ist der schnellhärtende Klebstoff durch eine äußere Energieeinbringung aushärtbar. Die Härtung des schnellhärtenden Klebstoffs wird durch Bestrahlung mit elektromagnetischer Strahlung - bevorzugt im Lichtwellenbereich - ausgelöst und bewirkt. Bekannt sind Klebstoffe, die bei Bestrahlung im UV-Wellenlängenbereich härten.

In diesem Zusammenhang ist es vorteilhaft, wenn das Anschlussgehäuse aus einem Material besteht, welches in demjenigen Wellenlängenbereich, in dem die für das Aushärten des schnellhärtenden Klebstoffes erforderliche Beleuchtung liegt, eine Transmission aufweist, die ausreichend hoch ist, um ein Aushärten des schnellhärtenden Klebstoffes bei einer gegebenen Beleuchtungsintensität in höchstens der vierfachen der für diese Beleuchtungsintensität angegebenen Zeitdauer zu erlauben. Auf diese Weise wird sichergestellt, dass der Klebstoff in ausreichend kurzer Zeit zuverlässig über die gesamte Fläche der Klebverbindung zwischen Hohlgehäuse und Anschlussgehäuse aushärtet.

In einer weiteren Ausführungsform wird das Aushärten des schnellhärtenden Klebstoffs durch äußere Energieeinwirkung lediglich aktiviert und die Aushärtung schreitet danach ohne diese Energieeinwirkung fort.

Dem Klebstoff können vorteilhafterweise Abstandsmittel zugefügt sein, um durch einen optimalen Klebespalt die Kapillarwirkung zu nutzen, die eine ausreichende und möglichst ganzflächige Benetzung zwischen den gegenüberliegenden Flächen des Anschlussgehäuses und des Hohlgehäuses bewirkt. Diese Abstandsmittel sind im Wesentlichen kugelförmig ausgebildet und weisen vorzugsweise einen Durchmesser von 0,1 mm bis 0,5 mm auf.

An Stelle von Epoxid kann grundsätzlich auch ein anderer, unter äußerer Energieeinwirkung härtender Klebstoff zum Verkleben es Hohlgehäuses mit dem Anschlusskörper verwendet werden. Aufgrund ihrer Eigenschaften besonders bevorzugt sind jedoch Epoxid und/oder Polyurethan.

Um eine zuverlässige und passgenaue Montage des vorgefertigten Anschlussgehäuses am Hohlgehäuse sicherzustellen, besitzt das Anschlussgehäuse auf seiner dem Hohlgehäuse zugewandten Seite vorzugsweise zwei Kavitäten, die der Aufnahme von an dem Hohlgehäuse befestigten Ankern in Form von vom Hohlgehäuse abstehenden Stiften aufweist, die zusammen mit den Kavitäten eine genaue Positionierung des Anschlussgehäuses insbesondere auch während des Verklebens des Anschlussgehäuses mit dem Hohlgehäuse bewirken und die Übertragung von Biegekräften zwischen Hohl- und Anschlussgehäuse verbessern.

Hinsichtlich Form und Volumen ist das Gehäuse vorzugsweise ein Gehäuse für einen Herzschrittmacher, einen Kardioverter/Defibrillator oder eine Kombination aus beiden. In diesem Sinne wird auch ein Herzschrittmacher oder Kardioverter/Defibrillator mit einem hier beanspruchten Gehäuse beansprucht.

Die Erfindung soll nun anhand eines Ausführungsbeispiels mit Bezug auf die Figuren näher erläutert werden. Von den Figuren zeigen:
- Figur 1:: ein Gehäuse eines Herzschrittmachers mit einem Hohlgehäuse aus biokompatiblem Metall und mit einem Anschlussgehäuse zum Anschließen von Elektrodenleitungen,
- Figur 2:: das Gehäuse aus Figur 1,
- Figur 3:: das Gehäuse aus Figur 1 mit einer Darstellung des Anschlussgehäuses mit Blick auf die dem Gehäuse zugewandten Seite,
- Figur 4:: das Gehäuse aus Figur 1 mit Verdrahtungselementen in halbtransparenter Darstellung.

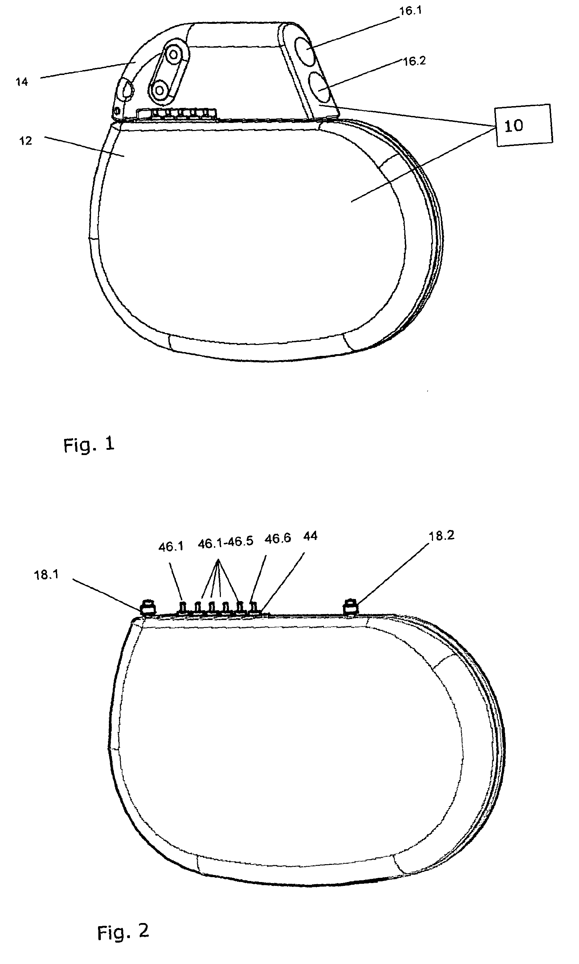

Figur 1 zeigt einen Herzschrittmacher mit einem Gehäuse 10, welches ein Hohlgehäuse 12 zur Aufnahme von Batterien, Kondensatoren und Steuerelektronik aufweist sowie ein Anschlussgehäuse 14 - auch Header genannt -, der zwei Steckbuchsen 16.1 und 16.2 aufweist, in denen Stecker von Elektrodenleitungen einzustecken sind.

Erfindungsgemäß ist das Anschlussgehäuse 14 mit dem Hohlgehäuse 12 verklebt. Dazu ist eine Klebeschicht aus Epoxid oder Polyurethan vorgesehen, die in Figur 1 nicht erkennbar ist und die sich zwischen den einander gegenüberliegenden Flächen des Anschlussgehäuses 14 und des Hohlgehäuses 12 befindet.

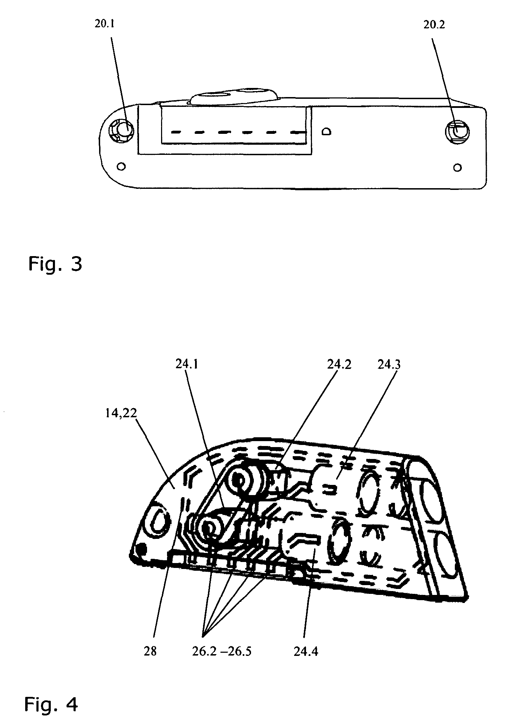

Um das Verkleben von Anschlussgehäuse 14 und Hohlgehäuse 12 zu erleichtern, sind am Hohlgehäuse 12 zwei Anker 18.1 und 18.2 vorgesehen (s. Figur 2), die in entsprechende, in Figur 3 erkennbare Kavitäten 20.1 und 20.2 des Anschlussgehäuses 14 eingreifen.

Wie aus Figur 4 zu entnehmen ist, enthält das transparente Anschlussgehäuse 14 darin angeordnete Kontaktbuchsen 24.1 bis 24.4, die die Steckbuchsen 16.1 und 16.2 teilweise umschließen, sowie elektrischen Zuleitungen 26 und eine Antenne 28. Außerdem sind in dem Anschlussgehäuse 14 Klemmschrauben zur Fixierung der Elektroden angeordnet, die mit Dichtungen abgedichtet sind. In einer weiteren Kavität des Anschlusskörpers 14 ist ein Röntgenmarker angeordnet.

Das Hohlgehäuse enthält eine Durchführung 44, mit der elektrische Anschlüsse aus dem Inneren des Hohlgehäuses 12 zu sechs in einer Reihe angeordneten elektrischen Kontaktstiften 46.1 bis 46.6 geführt sind. Die Kontaktstifte 46.1 und 46.6 dienen dabei der Kontaktierung der Antenne 28, während die Kontaktstifte 46.2 bis 46.5 der Kontaktierung der elektrischen Zuleitungen 26.1 bis 26.4 dienen, die zu den Kontaktbuchsen 24.1 bis 24.4 führen.

Die Kontaktstifte 46.1 bis 46.6 werden nach dem Aufsetzen des Anschlussgehäuses 14 auf das Hohlgehäuse 12 durch Verschweißen mit den elektrischen Zuleitungen 26.1 bis 26.4 sowie der Antenne 28 verbunden. Dazu ist auf der dem Hohlgehäuse 12 zugewandten Seite des Anschlussgehäuse 14 eine zunächst offene Kavität 46 vorgesehen, die nach dem Verschweißen der Kontaktstifte 46.1 bis 46.6 mit den Zuleitungen 26.1 bis 26.4 und der Antenne 28 vergossen wird.

Zur besseren Kennzeichnung des so entstandenen Gehäuses 10 kann ein individueller Röntgenmarker in die Kavität eingesetzt werden. Die Antenne 28 und die elektrischen Zuleitungen 26.1 und 26.4 werden mit den Kontaktstiften 46.1 bis 46.6 verschweißt. Nach Anschließen von Elektrodenleitungen an das Gehäuse 10 durch Einstecken von entsprechenden Elektrodenleitungssteckern in die Steckbuchsen 16.1 und 16.2 des Anschlussgehäuses 14 können die Elektrodenleitungsstecker mit Hilfe von Dichtungen und Klemmschrauben fixiert werden.

Ein geeignetes Material für das Anschlussgehäuse 14 ist transparentes Polyurethan, welches leicht vergossen werden kann und die gewünschten mechanischen und elektrischen sowie optischen Eigenschaften aufweist, nämlich transparent ist. Ein besonders geeignetes Polyurethan ist ein aromatisches Acryl-Polyesther Polyurethan. Weiterhin kann auch Epoxidharz verwendet werden.

Wie bereits erwähnt, ist ein geeigneter Klebstoff zum Verkleben des Anschlusskörpers 14 mit dem Hohlgehäuse 12 Epoxid oder auch Polyurethan. Besonders bevorzugt ist ein lichthärtendes Epoxid, welches bei Beleuchtung beziehungsweise Bestrahlung mit ultravioletter Strahlung aktiviert wird und aushärtet.

## Patentansprüche

1. Gehäuse (10) für ein medizinisches Implantat, wie einen Herzschrittmacher, Kardioverter/Defibrillator oder einer Kombination aus beidem, wobei
- das Gehäuse ein Hohlgehäuse (12) und einen an dem Hohlgehäuse (12) befestigten Anschlussgehäuse (14) aufweist, das wenigstens einen in einer von außen zugänglichen Kavität des Anschlussgehäuses angeordneten elektrischen Anschluss zum Anschließen einer Elektrodenleitung besitzt,
- das Material des Anschlussgehäuses (14) Kunststoff umfasst und das mit dem Hohlgehäuse (12) mittels eines Klebstoffs verklebt ist, und
- das Anschlussgehäuse (14) mit dem Hohlgehäuse (12) mittels eines Klebstoffs verklebt ist,
**dadurch gekennzeichnet, dass** der Kunststoff des Anschlussgehäuses (14) Polyurethan und/oder Polycarbonat oder Epoxidharz enthält und der Klebstoff schnellhärtend ist.

2. Gehäuse nach Anspruch 1, **dadurch gekennzeichnet, dass** der schnellhärtende Klebstoff 1 bis 15 Minuten, und bevorzugt 5 bis 10 Minuten, weiterverarbeitbar ist.

3. Gehäuse nach Anspruch 1 oder 2, **dadurch gekennzeichnet, dass** der schnellhärtende Klebstoff ein Epoxid oder Polyurethan ist.

4. Gehäuse nach einem der vorhergehenden Ansprüche 1 bis 3, **dadurch gekennzeichnet, dass** das Anschlussgehäuse (14) mehrteilig ist und seine Teilstücke mittels eines Klebstoffs verklebt sind.

5. Gehäuse nach Anspruch 1, **dadurch gekennzeichnet, dass** das Anschlussgehäuse (14) transparent ist und einen Blick von außerhalb der Anschlussgehäuses (14) auf den elektrischen Anschluss erlaubt.

6. Gehäuse nach Anspruch 5, **dadurch gekennzeichnet, dass** der transparente Kunststoff des Anschlussgehäuses (14) Polyurethan enthält.

7. Gehäuse nach einem der vorhergehenden Ansprüche 1 bis 6, **dadurch gekennzeichnet, dass** der Klebstoff unter Energieeinbringung aushärtet.

8. Gehäuse nach einem der vorhergehenden Ansprüche 1 bis 7, **dadurch gekennzeichnet, dass** der Klebstoff bei Temperaturen von ungefähr 20° bis ungefähr 85° Celsius aushärtet.

9. Gehäuse nach einem der vorhergehenden Ansprüche 1 bis 7, **dadurch gekennzeichnet, dass** der Klebstoff ein Klebstoff ist, der bei Bestrahlung mit elektromagnetischer Strahlung, bevorzugt im Lichtwellenlängenbereich aushärtet.

10. Gehäuse nach Anspruch 9, **dadurch gekennzeichnet, dass** der Kunststoff des Anschlussgehäuses (14) in demjenigen Wellenlängenbereich, der für das Aushärten des Klebstoffes erforderlich ist, eine Transmission aufweist, die ausreichend hoch ist, um ein Aushärten des Klebstoffes bei einer gegebenen Bestrahlungsintensität in höchstens der vierfachen der für diese Bestrahlungsintensität angegeben Zeitdauer zu erlauben.

11. Gehäuse nach einem der vorhergehenden Ansprüche 9 oder 10, **dadurch gekennzeichnet, dass** das Aushärten des Klebstoffs durch Energieeinwirkung aktiviert wird und danach selbständig fortschreitet.

12. Gehäuse nach einem der Ansprüche 1 bis 6, **dadurch gekennzeichnet, dass** dem Klebstoff Abstandsmittel in der Größe von ungefähr 0,1 mm bis 0,5 mm beigemengt sind.

13. Gehäuse nach einem der Ansprüche 1 bis 12, **dadurch gekennzeichnet, dass** das Anschlussgehäuse (14) auf seiner dem Hohlgehäuse (12) zugewandten Seite zwei Kavitäten (20) zur Aufnahme von an dem Hohlgehäuse befestigten Ankern (18) aufweist, die zusammen mit den Kavitäten (20) eine genaue Positionierung des Anschlussgehäuses (14) relativ zum Hohlgehäuse (12) vor und während des Verklebens des Anschlussgehäuses (14) mit dem Hohlgehäuse (12) bewirken.

14. Gehäuse nach einem der vorhergehenden Ansprüche 1 bis 12, **dadurch gekennzeichnet, dass** der elektrische Anschluss von wenigstens einer Steckbuchse (16) und einem in der Steckbuchse angeordneten elektrischen Kontakt (24) gebildet ist, wobei die Steckbuchse (16) zur Aufnahme eines Anschlusssteckers einer Elektrodenleitung ausgebildet ist.
